# FASCICULE DE BREVET EUROPEEN

(11) **EP 3 655 968 B1**
(45) Date de publication et mention de la délivrance du brevet: **28.10.2026**
(21) Numéro de dépôt: 18773513.9
(22) Date de dépôt: 13.07.2018
(51) Int. Cl.: G16H 40/63

(54) **SYSTEME AUTOMATISE DE REGULATION DE LA GLYCEMIE D'UN PATIENT**
AUTOMATISIERTES SYSTEM ZUR REGULIERUNG DES BLUTZUCKERSPIEGELS EINES PATIENTEN
AUTOMATED SYSTEM FOR REGULATING A PATIENT'S BLOOD GLUCOSE LEVEL

(30) Priorité: 21.07.2017 FR 1756960
(43) Date de publication de la demande: 27.05.2020
(73) Titulaire: Commissariat à l'Energie Atomique et aux Energies Alternatives, 75015 Paris (FR)
(72) Inventeur: DORON, Eléonore Maeva, 38700 La Tronche (FR); LACHAL, Sylvain, 38400 Saint Martin D'Heres (FR); JALLON, Pierre, 38700 Corenc (FR)
(74) Mandataire: Cabinet Beaumont
(86) Numéro de dépôt international: PCT/FR2018/051789
(87) Numéro de publication internationale: WO 2019/016452

(56) Documents cités:
- WO-A1-2010/025431
- GB-A- 2 436 873
- US-A1- 2008 183 060
- US-A1- 2014 066 884
- JALLON P ET AL: "Personalization of a compartmental physiological model for an artificial pancreas through integration of patient's state estimation", 2017 39TH ANNUAL INTERNATIONAL CONFERENCE OF THE IEEE ENGINEERING IN MEDICINE AND BIOLOGY SOCIETY (EMBC), IEEE, 11 July 2017 (2017-07-11), pages 1453 - 1456, XP033152274, DOI: 10.1109/EMBC.2017.8037108
- R. HOVORKA ET AL: "Blood Glucose Control by a Model Predictive Control Algorithm with Variable Sampling Rate Versus a Routine Glucose Management Protocol in Cardiac Surgery Patients: A Randomized Controlled Trial", JOURNAL OF CLINICAL ENDOCRINOLOGY & METABOLISM, vol. 92, no. 8, 1 August 2007 (2007-08-01), pages 2960 - 2964, XP055076241, ISSN: 0021-972X, DOI: 10.1210/jc.2007-0434
- PETR KOPECKÝ ET AL: "The Use of Continuous Glucose Monitoring Combined with Computer-Based eMPC Algorithm for Tight Glucose Control in Cardiosurgical ICU", BIOMED RESEARCH INTERNATIONAL, vol. 2013, 1 January 2013 (2013-01-01), pages 1 - 8, XP055458314, ISSN: 2314-6133, DOI: 10.1155/2013/186439
- ROMAN HOVORKA ET AL: "Nonlinear model predictive control of glucose concentration in subjects with type 1 diabetes", PHYSIOLOGICAL MEASUREMENT., vol. 25, no. 4, 23 July 2004 (2004-07-23), GB, pages 905 - 920, XP055420209, ISSN: 0967-3334, DOI: 10.1088/0967-3334/25/4/010
- SILVIA OVIEDO ET AL: "A review of personalized blood glucose prediction strategies for T1DM patients : Personalized Blood Glucose Prediction Strategies for T1D Patients", INTERNATIONAL JOURNAL FOR NUMERICAL METHODS IN BIOMEDICAL ENGINEERING, vol. 33, no. 6, 28 October 2016 (2016-10-28), pages e2833, XP055458095, ISSN: 2040-7939, DOI: 10.1002/cnm.2833

## Description

La présente demande de brevet revendique la priorité de la demande de brevet français FR17/56960.

### Domaine

La présente demande concerne le domaine des systèmes automatisés de régulation de glycémie, aussi appelés pancréas artificiels.

### Exposé de l'art antérieur

Un pancréas artificiel est un système permettant de réguler automatiquement les apports en insuline d'un patient diabétique à partir de son historique de glycémie, de son historique de prise de repas, et de son historique d'injection d'insuline.

On s'intéresse ici plus particulièrement aux systèmes de régulation de type MPC (de l'anglais "Model-based Predictive Control"), aussi appelés systèmes à commande prédictive, dans lesquels la régulation de la dose d'insuline administrée tient compte d'une prédiction de l'évolution future de la glycémie du patient, réalisée à partir d'un modèle physiologique décrivant l'assimilation de l'insuline par le corps du patient et son impact sur la glycémie du patient.

Il serait souhaitable de pouvoir améliorer les performances des pancréas artificiels à commande prédictive, et, plus particulièrement, de pouvoir améliorer la qualité de la prédiction de la glycémie future du patient, de façon à pouvoir contrôler avec une plus grande pertinence les apports en insuline et limiter les risques de placer le patient en situation d'hyperglycémie ou d'hypoglycémie.

Le document GB2436873 décrit des exemples de systèmes de régulation de glycémie.

### Résumé

L'invention est exposée dans le jeu de revendications annexé. Un mode de réalisation prévoit un système automatisé de régulation de la glycémie d'un patient selon la revendication 1.

Selon un mode de réalisation, l'indicateur numérique comprend l'écart quadratique moyen entre la glycémie estimée à partir du modèle et la glycémie réelle mesurée par le capteur pendant la période d'observation passée.

Selon un mode de réalisation, l'indicateur numérique comprend la différence entre la glycémie réelle mesurée par le capteur et la glycémie estimée par le modèle à un instant donné.

Selon un mode de réalisation, l'indicateur numérique comprend la différence entre la dérivée de la glycémie réelle mesurée par le capteur et la dérivée de la glycémie estimée par le modèle à un instant donné.

Selon un mode de réalisation, l'unité de traitement et de contrôle est configurée pour, à l'étape c), comparer la valeur de l'indicateur numérique à des premiers seuils, et sélectionner la durée de la période de prédiction parmi une pluralité de durées prédéfinies en fonction du résultat de la comparaison.

Selon un mode de réalisation, l'unité de traitement et de contrôle est en outre adaptée, après l'étape b), à déterminer, à partir de la valeur de l'indicateur numérique, si le modèle est suffisamment fiable pour pouvoir servir de base à la commande du dispositif d'injection d'insuline, et, dans le cas contraire, à commander le dispositif d'injection d'insuline selon une méthode de substitution, sans tenir compte de la prédiction réalisée à partir du modèle.

Selon un mode de réalisation, pour déterminer si le modèle est suffisamment fiable, l'unité de traitement et de contrôle compare la valeur de l'indicateur numérique à un deuxième seuil.

Selon un mode de réalisation, l'unité de traitement et de contrôle est adaptée à déterminer et ajuster automatiquement le deuxième seuil à partir de données passées mesurées sur le patient, de façon à ce que la commande du dispositif d'injection d'insuline soit basée sur les prédictions réalisées par le modèle au moins un certain pourcentage P du temps.

Selon un mode de réalisation, la méthode de substitution est une méthode de commande prédictive basée sur un modèle physiologique simplifié.

Selon un mode de réalisation, la méthode de substitution consiste à commander le dispositif d'injection d'insuline pour délivrer des doses d'insuline préprogrammées correspondant à un débit basal de référence prescrit au patient.

Selon un mode de réalisation, la méthode de substitution consiste à commander le dispositif d'injection d'insuline pour administrer des doses d'insuline déterminées par l'unité de traitement et de contrôle en fonction du niveau courant de glycémie mesuré par le capteur et/ou de la vitesse de variation de la glycémie mesurée par le capteur.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 représente de façon schématique, sous forme de blocs, un exemple d'un mode de réalisation d'un système automatisé de régulation de la glycémie d'un patient ;
la figure 2 est une représentation simplifiée d'un modèle physiologique utilisé dans le système de la figure 1 pour prédire l'évolution future de la glycémie du patient ;
la figure 3 est un diagramme illustrant un exemple d'un procédé automatisé de régulation de glycémie pouvant être mis en oeuvre par le système de la figure 1 ; et
la figure 4 est un diagramme illustrant plus en détail un exemple d'un mode de réalisation d'un procédé automatisé de régulation de glycémie mis en oeuvre par le système de la figure 1.

### Description détaillée

De mêmes éléments ont été désignés par de mêmes références dans les différentes figures. Par souci de clarté, seuls les éléments qui sont utiles à la compréhension des modes de réalisation décrits ont été représentés et sont détaillés. En particulier, le dispositif de mesure de glycémie et le dispositif d'injection d'insuline du système de régulation décrit n'ont pas été détaillés, les modes de réalisation décrits étant compatibles avec tous ou la plupart des dispositifs de mesure de glycémie et d'injection d'insuline connus. De plus, la réalisation matérielle de l'unité de traitement et de contrôle du système de régulation décrit n'a pas été détaillée, la réalisation d'une telle unité de traitement et de contrôle étant à la portée de l'homme du métier à partir des indications fonctionnelles de la présente description.

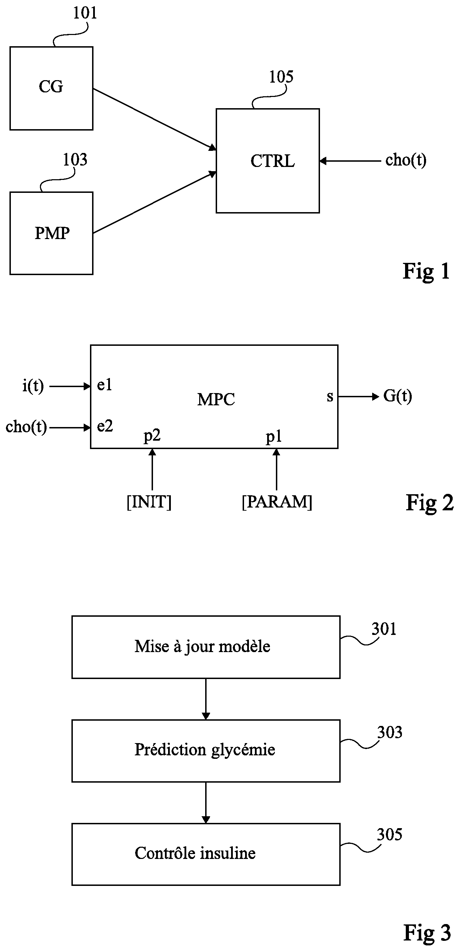

La figure 1 représente de façon schématique, sous forme de blocs, un exemple d'un mode de réalisation d'un système automatisé de régulation de la glycémie d'un patient.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu (par exemple au moins une fois toutes les cinq minutes) la glycémie du patient. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés. Dans cet exemple, l'unité de traitement et de contrôle 105 est en outre adaptée à recevoir, par l'intermédiaire d'une interface utilisateur non détaillée, des données cho(t) représentatives de l'évolution, en fonction du temps, de la quantité de glucose ingérée par le patient. L'interface utilisateur peut en outre être prévue pour permettre de saisir des informations supplémentaires susceptibles de faciliter la régulation de la glycémie, par exemple des informations relatives à l'activité physique du patient, ou encore à son stress, ou toute autre information relative au métabolisme du patient, ou encore les types d'aliments ingérés par le patient (gras ou non par exemple).

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient (ainsi que des éventuelles informations supplémentaires susmentionnées). Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile transporté par le patient tout au long de la journée et/ou de la nuit, par exemple un dispositif de type smartphone configuré pour mettre en oeuvre un procédé de régulation du type décrit ci-après.

Dans le mode de réalisation de la figure 1, l'unité de traitement et de contrôle 105 est adaptée à déterminer la quantité d'insuline à administrer au patient en tenant compte d'une prédiction de l'évolution future de sa glycémie en fonction du temps. Plus particulièrement, l'unité de traitement et de contrôle 105 est adaptée, à partir de l'historique d'insuline injectée et de l'historique de glucose ingéré (ainsi que des éventuelles informations supplémentaires susmentionnées), et en se basant sur un modèle physiologique décrivant l'assimilation de l'insuline par le corps du patient et son impact sur la glycémie, à déterminer une courbe représentative de l'évolution attendue de la glycémie du patient en fonction du temps, sur une période à venir appelée période de prédiction ou horizon de prédiction, par exemple une période de 1 à 10 heures. En tenant compte de cette courbe, l'unité de traitement et de contrôle 105 détermine les doses d'insuline qu'il conviendrait d'injecter au patient pendant la période de prédiction à venir, pour que la glycémie réelle (par opposition à la glycémie estimée à partir du modèle physiologique) du patient reste dans des limites acceptables, et en particulier pour limiter les risques d'hyperglycémie ou d'hypoglycémie. Dans ce mode de fonctionnement, comme cela sera expliqué plus en détail ci-après, les données de glycémie réelle mesurées par le capteur 101 sont utilisées principalement à des fins de calibration du modèle physiologique.

La figure 2 est une représentation simplifiée d'un modèle physiologique MPC utilisé dans le système de la figure 1 pour prédire l'évolution future de la glycémie du patient. Sur la figure 2, le modèle est représenté sous la forme d'un bloc de traitement comportant :
une entrée e1 sur laquelle est appliquée un signal i(t) représentatif de l'évolution, en fonction du temps t, de la quantité d'insuline injectée au patient ;
une entrée e2 sur laquelle est appliquée un signal cho(t) représentatif de l'évolution, en fonction du temps t, de la quantité de glucose ingérée par le patient ; et
une sortie s fournissant un signal G(t) représentatif de l'évolution, en fonction du temps t, de la glycémie du patient.

Le modèle physiologique MPC est par exemple un modèle compartimental comportant, outre les variables d'entrée i(t) et cho(t) et la variable de sortie G(t), une pluralité de variables d'états correspondant à des variables physiologiques du patient, évoluant en fonction du temps. L'évolution temporelle des variables d'état et de la variable de sortie G(t) est régie par un système d'équations différentielles comportant une pluralité de paramètres représentés sur la figure 2 par un vecteur [PARAM] appliqué sur une entrée p1 du bloc MPC. La réponse du modèle physiologique est en outre conditionnée par les états initiaux ou valeurs initiales affectés aux variables d'état, représentés sur la figure 2 par un vecteur [INIT] appliqué sur une entrée p2 du bloc MPC.

A titre d'exemple, le modèle physiologique MPC utilisé dans le système de la figure 1 est le modèle dit de Hovorka, décrit dans l'article intitulé "Nonlinear model predictive control of glucose concentration in subjects with type 1 diabetes" de Roman Hovorka et al. (Physiol Meas. 2004;25:905-920), et dans l'article intitulé "Partitioning glucose distribution/transport, disposal, and endogenous production during IVGTT", de Roman Hovorka et al. (Am J Physiol Endocrinol Metab 282: E992-E1007, 2002). Plus généralement, tout autre modèle physiologique décrivant l'assimilation de l'insuline par le corps d'un patient et son effet sur la glycémie du patient peut être utilisé, par exemple le modèle dit de Cobelli, décrit dans l'article intitulé "A System Model of Oral Glucose Absorption: Validation on Gold Standard Data", de Chiara Dalla Man et al. (IEEE TRANSACTIONS ON BIOMEDICAL ENGINEERING, VOL. 53, NO. 12, DECEMBER 2006).

Parmi les paramètres du vecteur [PARAM], certains peuvent être considérés comme constants pour un patient donné. D'autres paramètres, appelés ci-après paramètres temps-dépendants, sont en revanche susceptibles d'évoluer dans le temps. Du fait de cette variabilité de certains paramètres du système, il est en pratique nécessaire de ré-étalonner ou re-calibrer régulièrement le modèle en cours d'utilisation, par exemple toutes les 1 à 20 minutes, par exemple toutes les 5 minutes, pour s'assurer que les prédictions du modèle restent pertinentes. Cette mise à jour du modèle, aussi appelée personnalisation du modèle, doit pouvoir être réalisée de façon automatique par le système de la figure 1, c'est-à-dire sans qu'il soit nécessaire de mesurer physiquement les paramètres temps-dépendants du système sur le patient puis de les transmettre à l'unité de traitement et de contrôle 105.

La figure 3 est un diagramme illustrant un exemple d'un procédé automatisé de régulation de glycémie pouvant être mis en oeuvre par le système de la figure 1.

Ce procédé comprend une étape 301 de re-calibration ou mise à jour du modèle, pouvant par exemple être répétée à intervalles réguliers, par exemple toutes les 1 à 20 minutes. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée de durée ΔT, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. A titre d'exemple, l'unité de traitement et de contrôle recherche un jeu de paramètres conduisant à minimiser un indicateur m représentatif de l'aire entre la courbe de glycémie estimée par le modèle et la courbe de glycémie réelle pendant la période d'observation, aussi appelé écart quadratique moyen entre la glycémie estimée et la glycémie réelle, par exemple défini comme suit : $m = \frac{1}{Δ T} {\sum }_{t = {t}_{0} - Δ T}^{{t}_{0}} {\left|g\left(t\right)-\hat{g}\left(t\right)\right|}^{2}$ où t est la variable temps discrétisée, t₀-*Δ*T correspond à l'instant de début de la phase d'observation passé, t₀ correspond à l'instant de fin de la phase d'observation passée (correspondant par exemple à l'instant de début de l'étape de calibration du modèle), g est la courbe d'évolution temporelle de la glycémie réelle mesurée par le capteur 101 pendant la période [t₀-*Δ*T, t₀], et ***ĝ*** est la courbe de glycémie estimée à partir du modèle pendant la période [t₀-*Δ*T, t₀]. A titre de variante, pour le calcul de l'écart quadratique moyen, la variable ΔT peut être remplacée par le nombre de mesures réalisées pendant la période d'observation passée. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût.

On notera que lors de l'étape 301, outre les paramètres temps-dépendants du modèle, l'unité de traitement et de contrôle 105 définit un vecteur [INIT] d'états initiaux (états à l'instant t₀-*Δ*T) des variables d'état du modèle, pour pouvoir simuler le comportement du patient à partir du modèle. Pour définir les états initiaux des variables d'état du modèle, une première possibilité consiste à faire l'hypothèse que, dans la période précédant la période d'observation [t₀-*Δ*T, t₀] sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial t₀-*Δ*T. Les valeurs à l'instant t₀-*Δ*T des variables d'état du système peuvent alors être calculées analytiquement. Pour améliorer l'initialisation, une autre possibilité consiste à faire les mêmes hypothèses que précédemment, mais en ajoutant la contrainte que la glycémie estimée à l'instant t₀-*Δ*T soit égale à la glycémie réelle mesurée par le capteur. Pour améliorer encore l'initialisation, une autre possibilité est de considérer les états initiaux des variables d'état du modèle comme des variables aléatoires, au même titre que les paramètres temps-dépendants du modèle. Les états initiaux des variables d'état sont alors déterminés de la même façon que les paramètres temps-dépendants du modèle, c'est-à-dire que l'unité de traitement et de contrôle 105 recherche un jeu de valeurs d'états initiaux [INIT] conduisant à minimiser une grandeur représentative de l'erreur entre la courbe de glycémie estimée par le modèle et la courbe de glycémie réelle pendant la période d'observation passée.

Le procédé de la figure 3 comprend en outre, après l'étape 301, une étape 303 de prédiction, par l'unité de traitement et de contrôle 105, de l'évolution temporelle de la glycémie du patient sur une période de prédiction à venir [t0, t₀+T_{pred}] de durée T_{pred}, par exemple comprise entre 1 et 10 heures, à partir du modèle physiologique mis à jour à l'étape 301 et en tenant compte de l'historique d'insuline injectée au patient et de l'historique de glucose ingéré par le patient.

Le procédé de la figure 3 comprend de plus, après l'étape 303, une étape 305 de détermination, par l'unité de traitement et de contrôle 105, en tenant compte de la courbe de glycémie future prédite à l'étape 303, des doses d'insuline à injecter au patient pendant la période de prédiction à venir [t0, t₀+T_{pred}]. A l'issue de cette étape, l'unité de traitement et de contrôle 105 peut programmer le dispositif d'injection 103 pour administrer les doses déterminées pendant la période de prédiction [t0, t₀+T_{pred}].

Les étapes 303 de prédiction de la glycémie et 305 et détermination des doses futures d'insuline à administrer peuvent par exemple être réitérées à chaque mise à jour du modèle physiologique (c'est-à-dire après chaque itération de l'étape 301), à chaque nouvelle ingestion de glucose signalée par le patient, et/ou à chaque nouvelle administration d'une dose d'insuline par le dispositif d'injection 103.

Dans le procédé susmentionné, la durée T_{pred} de la période de prédiction de l'évolution future de la glycémie du patient est un paramètre important, conditionnant la performance du système de régulation. Compte tenu de la dynamique relativement lente du système que l'on cherche à réguler, il serait souhaitable que la période de prédiction T_{pred} soit relativement longue, par exemple de l'ordre de 4 heures ou plus, de façon à pouvoir anticiper et évaluer au mieux les besoins en insuline du patient. Toutefois, en pratique, les imperfections du modèle utilisé contraignent à limiter l'horizon de prédiction considéré.

Selon un aspect d'un mode de réalisation, l'unité de traitement et de contrôle 105 est adaptée, après chaque mise à jour du modèle physiologique (étape 301), à calculer un ou plusieurs indicateurs numériques représentatifs de la fiabilité du modèle mis à jour, et à ajuster la durée de prédiction T_{pred} en fonction de ces indicateurs. Plus particulièrement, si le modèle mis à jour est jugé fiable, la durée de prédiction T_{pred} sera choisie relativement élevée, et, si le modèle est jugé peu fiable, la durée de prédiction T_{pred} sera choisie relativement faible. Par rapport à un système dans lequel la durée de prédiction T_{pred} est fixe, un avantage de ce mode de fonctionnement est qu'il permet d'améliorer la qualité de la prédiction de la glycémie future du patient, et ainsi de contrôler avec une plus grande pertinence les apports en insuline.

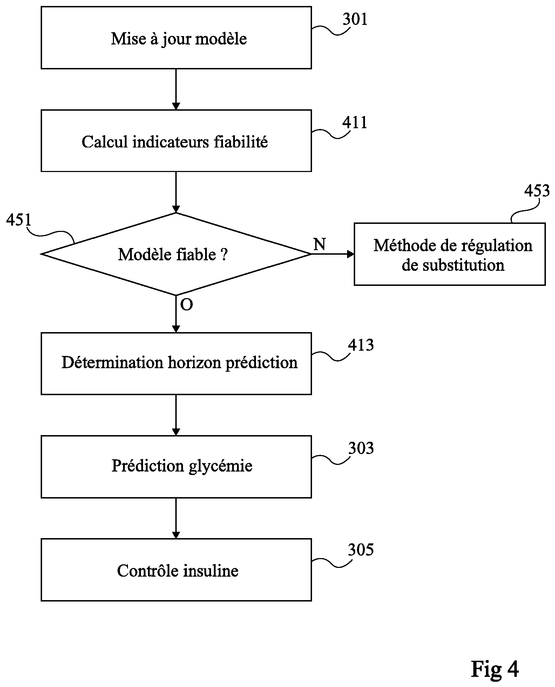

La figure 4 est un diagramme illustrant plus en détail un exemple d'un procédé automatisé de régulation de glycémie mis en oeuvre par le système de la figure 1, dans lequel la durée de prédiction T_{pred} est ajustée en fonction d'une estimation de la fiabilité du modèle physiologique.

Ce procédé comprend les mêmes étapes 301, 303 et 305 que dans l'exemple de la figure 3. Toutefois, le procédé de la figure 4 comprend en outre, après chaque étape 301 de mise à jour du modèle physiologique et avant la mise en oeuvre des étapes suivantes 303 de prédiction de la glycémie future du patient, et 305 de contrôle de la délivrance d'insuline à partir de la prédiction de glycémie, une étape 411 de calcul d'un ou plusieurs indicateurs numériques de fiabilité du modèle mis à jour, et d'une étape 413 d'ajustement de la durée de prédiction T_{pred} en fonction du ou des indicateurs de fiabilité calculés à l'étape 411.

Lors de l'étape 411, l'unité de traitement et de contrôle 105 calcule un ou plusieurs indicateurs numériques représentatifs de la fiabilité du modèle mis à jour à l'étape 301. A titre d'exemple, l'unité de traitement et de contrôle calcule trois indicateurs numériques de fiabilité MM, GD et SD. L'indicateur MM correspond à l'écart quadratique moyen entre la glycémie estimée à partir du modèle mis à jour et la courbe de glycémie réelle mesurée par le capteur 101 pendant une période d'observation passée, par exemple une période de 1 à 10 heures précédant l'instant t₀, par exemple la période [t₀-*Δ*T, t₀]. L'indicateur GD correspond à la différence entre la glycémie réelle mesurée par le capteur 101 et la glycémie estimée par le modèle mis à jour à un instant donné, par exemple à l'instant t₀, et l'indicateur SD correspond à la différence entre la pente ou dérivée de la glycémie réelle mesurée par le capteur 101 et la pente ou dérivée de la glycémie estimée par le modèle mis à jour à un instant donné, par exemple à l'instant t0.

Lors de l'étape 413, l'unité de traitement et de contrôle détermine, à partir du ou des indicateurs numériques de fiabilité calculés à l'étape 411, la durée de prédiction T_{pred} à utiliser pour la mise en oeuvre de l'étape 303. A titre d'exemple, la durée de prédiction T_{pred} est choisie parmi n valeurs prédéfinies D1, ..., Dn décroissantes, avec n entier supérieur ou égal à 2, en fonction de la valeur du ou des indicateurs numériques de fiabilité calculés à l'étape 411. A titre d'exemple, pour chacun des m indicateurs de fiabilité Iⱼ calculé à l'étape 411, avec j entier allant de 1 à m et m entier supérieur ou égal à 1, la valeur de l'indicateur est comparée à un jeu de n seuils prédéfinis SIⱼ₁, ..., SIⱼₙ de valeurs croissantes. L'unité de traitement et de contrôle 105 recherche alors le plus petit indice de seuil k tel que, pour chacun des m indicateurs Iⱼ calculés à l'étape 411, la valeur de l'indicateur Iⱼ soit inférieure au seuil SIⱼₖ. L'horizon de prédiction T_{pred} est alors choisi égal à la durée Dk. Ainsi, la durée T_{pred} de la période de prédiction à venir est choisie d'autant plus courte que l'erreur entre la glycémie estimée à partir du modèle et la glycémie réelle mesurée par le capteur sur la période d'observation passée est importante, et inversement. Autrement dit, la durée T_{pred} de la période de prédiction à venir est une fonction décroissante de l'erreur entre la glycémie estimée à partir du modèle et la glycémie réelle mesurée par le capteur sur la période d'observation passée, étant entendu que par fonction décroissante on entend ici une fonction qui peut être décroissante de façon continue, ou décroissante par paliers. Plus généralement, selon l'objectif recherché, d'autres fonctions et/ou règles de décision permettant de déterminer la durée de prédiction T_{pred} à partir du ou des indicateurs de fiabilité calculés à l'étape 411 peuvent être implémentées.

Après l'étape 413, les étapes 303 et 305 peuvent être mises en oeuvre de façon similaire à ce qui a été décrit précédemment.

On notera que dans certains cas, la fiabilité du modèle physiologique mis à jour à l'étape 301 peut être si faible qu'il est préférable de cesser d'utiliser le modèle pour réguler la glycémie du patient.

Dans l'exemple de la figure 4, l'unité de contrôle et de traitement 105 du système de régulation est en outre adaptée, après chaque mise à jour ou re-calibration du modèle physiologique (étape 301), à partir du ou des indicateurs de fiabilité calculés à l'étape 411, à déterminer si le modèle mis à jour est suffisamment fiable pour pouvoir être utilisé pour réguler la glycémie du patient.

Plus particulièrement, le procédé de la figure 4 comprend, entre les étapes 411 et 413, une étape 451 de vérification de la fiabilité du modèle mis à jour à l'étape 301. A titre d'exemple, la fiabilité du modèle peut être considérée comme suffisante par l'unité de traitement et de contrôle 105 lorsque les valeurs des indicateurs calculés à l'étape 411 sont inférieures à des seuils prédéfinis, et insuffisante dans le cas contraire. A titre d'exemple, en reprenant les notations définies ci-dessus, la fiabilité du modèle peut être considérée comme suffisante par l'unité de traitement et de contrôle 105 lorsque pour chacun des m indicateurs de fiabilité Iⱼ calculés à l'étape 411, la valeur de l'indicateur est inférieure au seuil SIⱼₙ correspondant, et insuffisante lorsque pour au moins l'un des indicateurs Iⱼ, la valeur de l'indicateur est supérieure au seuil SIⱼₙ correspondant. Plus généralement, tout autre critère de qualité ou toute autre combinaison de critères de qualité peuvent être utilisés à l'étape 451 pour déterminer si le modèle physiologique re-calibré à l'étape 301 est suffisamment fiable.

Si le modèle physiologique est considéré comme suffisamment fiable à l'étape 451 (O), les étapes 413, 303 et 305 peuvent être mises en oeuvre de façon similaire à ce qui a été décrit précédemment, c'est-à-dire que l'unité de traitement et de contrôle 105 continue de se baser sur les prédictions réalisées par le modèle physiologique pour réguler l'administration de l'insuline au patient, en ajustant l'horizon de prédiction T_{pred} en fonction du degré de fiabilité du modèle.

Si le modèle physiologique est jugé insuffisamment fiable à l'étape 451 (N), l'unité de traitement et de contrôle 105 cesse d'utiliser ce modèle pour réguler l'administration de l'insuline au patient, et met en oeuvre une méthode de régulation de substitution lors d'une étape 453.

A titre d'exemple, lors de l'étape 453, l'unité de traitement et de contrôle 105 utilise un modèle physiologique simplifié, par exemple un modèle compartimental comportant un nombre de variables d'état et un nombre de paramètres réduit par rapport au modèle initial, pour prédire l'évolution de la glycémie du patient et réguler en conséquence l'injection d'insuline.

A titre de variante, lors de l'étape 453, l'unité de traitement et de contrôle 105 cesse de mettre en oeuvre une commande prédictive, c'est-à-dire qu'elle cesse d'utiliser un modèle physiologique pour prédire la glycémie future du patient et réguler en conséquence l'injection d'insuline. Dans ce cas, l'unité de traitement et de contrôle 105 commande par exemple le dispositif d'injection d'insuline 103 pour administrer des doses préprogrammées d'insuline, correspondant par exemple à un débit basal de référence prescrit au patient. Alternativement, l'unité de traitement et de contrôle 105 utilise un algorithme de type matrice décisionnelle pour déterminer les doses d'insuline à administrer au patient, en fonction de divers paramètres observés tels que le niveau courant de glycémie mesuré par le capteur 101, ou encore la vitesse de variation (ou pente) de la glycémie sur une période passée.

Une telle méthode de substitution peut par exemple être utilisée pendant une période de temps prédéterminée. A l'issue de cette période, les étapes 301 de calibration du modèle physiologique principal, 411 de calcul du ou des indicateurs de fiabilité du modèle physiologique principal, et 451 d'estimation de la qualité du modèle physiologique principal, peuvent être réitérées, pour, si la qualité du modèle physiologique principal est jugée suffisante, réactiver l'utilisation du modèle principal pour réguler l'administration de l'insuline au patient.

A titre d'exemple, les seuils utilisés à l'étape 451 pour déterminer si le modèle physiologique principal est suffisamment fiable pour pouvoir être utilisé sont choisis de façon à maximiser la probabilité que le système de régulation fonctionne au moins un certain pourcentage P du temps, par exemple au moins 70% du temps, sur la base du modèle physiologique principal.

Les seuils utilisés aux étapes 451 et 413 sont par exemples déterminés sur la base d'une analyse de données passées mesurée sur un échantillon de plusieurs patients. A titre d'exemple, on peut rejouer l'algorithme de régulation pour une pluralité de patients sur un banc de test, et, pour chaque patient, à chaque mise à jour du modèle physiologique, pour chacune des n valeurs possibles D1, ..., Dn de la durée de prédiction T_{pred}, calculer l'écart quadratique moyen, sur la période de prédiction T_{pred}, entre la glycémie estimée à partir du modèle mis à jour et la courbe de glycémie réelle mesurée par le capteur 101. A chaque mise à jour du modèle, on calcule en outre les m indicateurs de fiabilité du modèle mis à jour I₁, ..., Iₘ. Ainsi, à chaque mise à jour du modèle, on dispose d'un jeu de m valeurs correspondant aux indicateurs de fiabilité du modèle tels que défini ci-dessus, et d'un jeu de n valeurs correspondant à des mesures effectives de fiabilité du modèle pour les n durées de prédiction D1, ..., Dn considérées. L'étude des corrélations entre les indicateurs de fiabilité du modèle et les mesures effectives de fiabilité permet de déterminer les seuils à utiliser à l'étape 413 pour choisir la durée de la période de prédiction après chaque mise à jour du modèle, et/ou à l'étape 451 pour décider s'il convient ou non de basculer sur une méthode de régulation de substitution. La détermination des seuils à partir des valeurs susmentionnées d'indicateurs de fiabilité et de mesures effectives de fiabilité peut être totalement ou partiellement automatisée.

A titre de variante, les seuils utilisés aux étapes 451 et 413 sont déterminés de façon similaire à ce qui vient d'être décrit, mais uniquement sur la base des données passées mesurées sur le patient utilisateur du système, ce qui permet de personnaliser le fonctionnement du système de régulation. Dans ce cas, l'unité de traitement et de contrôle 105 peut être configurée pour recalculer régulièrement les seuils utilisés à l'étape 413 et/ou à l'étape 451, en tenant compte des nouvelles données mesurées sur le patient depuis la dernière mise à jour des seuils.

## Revendications

1. Système automatisé de régulation de la glycémie d'un patient, comportant :
un capteur de glycémie (101) ;
un dispositif d'injection d'insuline (103) ; et
une unité de traitement et de contrôle (105),
dans lequel l'unité de traitement et de contrôle (105) est adaptée à :
a) mettre en oeuvre, en tenant compte de la glycémie mesurée par le capteur (101) au cours d'une période d'observation passée, une étape de calibration automatique d'un modèle physiologique adapté à prédire l'évolution de la glycémie du patient ;
b) à l'issue de l'étape de calibration, calculer au moins un indicateur numérique représentatif de l'erreur entre la glycémie estimée à partir du modèle et la glycémie réelle mesurée par le capteur sur la période d'observation passée ;
c) comparer la valeur dudit au moins un indicateur numérique à des seuils prédéfinis, et déterminer la durée d'une période de prédiction à venir en tenant compte du résultat de la comparaison, la durée de la période de prédiction à venir étant choisie d'autant plus courte que l'erreur entre la glycémie estimée à partir du modèle et la glycémie réelle mesurée par le capteur sur la période d'observation passée est importante, et inversement ; et
d) prédire, à partir du modèle physiologique, l'évolution future de la glycémie du patient sur la période de prédiction à venir, et commander le dispositif d'injection d'insuline (103) en tenant compte de cette prédiction,
dans lequel les étapes a) à d) sont répétées à intervalles réguliers,
dans lequel l'unité de traitement et de contrôle (105) est configurée pour répéter les étapes a) à d) toutes les 1 à 20 minutes, et dans lequel la période de prédiction à venir déterminée à l'étape c) est comprise entre 1 et 10 heures.

2. Système selon la revendication 1, dans lequel ledit au moins un indicateur numérique comprend l'écart quadratique moyen entre la glycémie estimée à partir du modèle et la glycémie réelle mesurée par le capteur (101) pendant la période d'observation passée.

3. Système selon la revendication 1 ou 2, dans lequel ledit au moins un indicateur numérique comprend la différence entre la glycémie réelle mesurée par le capteur (101) et la glycémie estimée par le modèle à un instant donné.

4. Système selon l'une quelconque des revendications 1 à 3, dans lequel ledit au moins un indicateur numérique comprend la différence entre la dérivée de la glycémie réelle mesurée par le capteur (101) et la dérivée de la glycémie estimée par le modèle à un instant donné.

5. Système selon l'une quelconque des revendications 1 à 4, dans lequel l'unité de traitement et de contrôle (105) est configurée pour, à l'étape c), comparer la valeur dudit au moins un indicateur numérique à des premiers seuils, et sélectionner la durée de la période de prédiction parmi une pluralité de durées prédéfinies en fonction du résultat de la comparaison.

6. Système selon l'une quelconque des revendications 1 à 5, dans lequel l'unité de traitement et de contrôle (105) est en outre adaptée, après l'étape b), à déterminer, à partir de la valeur dudit au moins un indicateur numérique, si le modèle est suffisamment fiable pour pouvoir servir de base à la commande du dispositif d'injection d'insuline, et, dans le cas contraire, à commander le dispositif d'injection d'insuline (103) selon une méthode de substitution, sans tenir compte de la prédiction réalisée à partir du modèle.

7. Système selon la revendication 6, dans lequel l'unité de traitement et de contrôle (105) est configurée pour, pour déterminer si le modèle est suffisamment fiable, comparer la valeur dudit au moins un indicateur numérique à un deuxième seuil.

8. Système selon la revendication 7, dans lequel l'unité de traitement et de contrôle (105) est adaptée à déterminer et ajuster automatiquement le deuxième seuil à partir de données passées mesurées sur le patient, de façon à ce que la commande du dispositif d'injection d'insuline (103) soit basée sur les prédictions réalisées par le modèle au moins un certain pourcentage du temps.

9. Système selon l'une quelconque des revendications 6 à 8, dans lequel la méthode de substitution est une méthode de commande prédictive basée sur un modèle physiologique simplifié.

10. Système selon l'une quelconque des revendications 6 à 8, dans lequel la méthode de substitution consiste à commander le dispositif d'injection d'insuline (103) pour délivrer des doses d'insuline préprogrammées correspondant à un débit basal de référence prescrit au patient.

11. Système selon l'une quelconque des revendications 6 à 8, dans lequel la méthode de substitution consiste à commander le dispositif d'injection d'insuline (103) pour administrer des doses d'insuline déterminées par l'unité de traitement et de contrôle (105) en fonction du niveau courant de glycémie mesuré par le capteur (101) et/ou de la vitesse de variation de la glycémie mesurée par le capteur (101).

## Patentansprüche

1. Ein automatisiertes System zur Regulierung des Blutzuckers eines Patienten, das Folgendes aufweist:
einen Blutzuckersensor (101);
eine Insulininjektionseinrichtung (103); und
eine Verarbeitungs- und Steuereinheit (105),
wobei die Verarbeitungs- und Steuereinheit (105) zu Folgendem in der Lage ist:
a) Durchführen, unter Berücksichtigung des Blutzuckers, der während eines vergangenen Beobachtungszeitraums durch den Sensor (101) gemessen wurde, eines Schritts der automatisierten Kalibrierung eines physiologischen Modells, das zum Vorhersagen der Entwicklung des Blutzuckers des Patienten in der Lage ist;
b) am Ende des Kalibrierungsschritts Berechnen wenigstens eines numerischen Indikators, der für den Fehler zwischen dem aus dem Modell geschätzten Blutzucker und dem durch den Sensor über den vergangenen Beobachtungszeitraum gemessenen tatsächlichen Blutzucker repräsentativ ist;
c) Vergleichen des Werts des genannten numerischen Indikators mit vordefinierten Schwellenwerten und Bestimmen der Dauer eines kommenden Vorhersagezeitraums unter Berücksichtigung des Werts des Ergebnisses des genannten Vergleichs, wobei die Dauer des kommenden Vorhersagezeitraums so viel kürzer ausgewählt wird, je signifikanter der Fehler zwischen dem aus dem Modell geschätzten Blutzucker und dem durch den Sensor über den vergangenen Beobachtungszeitraum gemessenen tatsächlichen Blutzucker ist, und umgekehrt; und
d) Vorhersagen, ausgehend von dem physiologischen Modell, der zukünftigen Entwicklung des Blutzuckers des Patienten über den kommenden Vorhersagezeitraum und Steuern der Insulininjektionseinrichtung (103) unter Berücksichtigung der Vorhersage,
wobei die Schritte a) bis d) in regelmäßigen Intervallen wiederholt werden,
wobei die Verarbeitungs- und Steuereinheit (105) eingerichtet ist, die Schritte (a) bis (d) alle 1 bis 20 Minuten zu wiederholen, und wobei der in Schritt (c) bestimmte zukünftige Vorhersagezeitraum zwischen 1 und 10 Stunden liegt.

2. Das System nach Anspruch 1, wobei der genannte wenigstens eine numerische Indikator die Standardabweichung zwischen dem aus dem Modell geschätzten Blutzucker und dem tatsächlichen Blutzucker, der während des vergangenen Beobachtungszeitraums durch den Sensor (101) gemessen wurde, aufweist.

3. Das System nach Anspruch 1 oder 2, wobei der genannte wenigstens eine numerische Indikator die Differenz zwischen dem tatsächlichen Blutzucker, der durch den Sensor (101) gemessen wird, und dem Blutzucker, der durch das Modell zu einem gegebenen Zeitpunkt geschätzt wird, aufweist.

4. Das System nach einem der Ansprüche 1 bis 3, wobei der genannte wenigstens eine numerische Indikator die Differenz zwischen der Ableitung des tatsächlichen Blutzuckers, der durch den Sensor (101) gemessen wird, und der Ableitung des Blutzuckers, der durch das Modell zu einem gegebenen Zeitpunkt geschätzt wird, aufweist.

5. Das System nach einem der Ansprüche 1 bis 4, wobei die Verarbeitungs- und Steuereinheit (105) eingerichtet ist, in Schritt c) den Wert des genannten wenigstens einen numerischen Indikators mit ersten Schwellenwerten zu vergleichen und die Dauer des Vorhersagezeitraums aus einer Vielzahl vordefinierter Dauern nach dem Ergebnis des Vergleichs auszuwählen.

6. Das System nach einem der Ansprüche 1 bis 5, wobei die Verarbeitungs- und Steuereinheit (105) ferner in der Lage ist, nach Schritt b) auf der Grundlage des Werts des genannten wenigstens einen numerischen Indikators zu bestimmen, ob das Modell ausreichend zuverlässig ist, um als Grundlage für die Steuerung der Insulininjektionseinrichtung verwendet zu werden, und im gegenteiligen Fall die Insulininjektionseinrichtung (103) nach einem Ersatzverfahren zu steuern, ohne die ausgehend von dem Modell durchgeführte Vorhersage zu berücksichtigen.

7. Das System nach Anspruch 6, wobei die Verarbeitungs- und Steuereinheit (105) eingerichtet ist, zum Bestimmen, ob das Modell ausreichend zuverlässig ist, den Wert des genannten wenigstens einen numerischen Indikators mit einem zweiten Schwellenwert zu vergleichen.

8. Das System nach Anspruch 7, wobei die Verarbeitungs- und Steuereinheit (105) in der Lage ist, den zweiten Schwellenwert aus vergangenen, an dem Patienten gemessenen Daten zu bestimmen und automatisch anzupassen, sodass die Steuerung der Insulininjektionseinrichtung (103) wenigstens für einen bestimmten Prozentsatz der Zeit auf den durch das Modell vorgenommenen Vorhersagen basiert.

9. Das System nach einem der Ansprüche 6 bis 8, wobei das Ersatzverfahren ein prädiktives Steuerverfahren ist, das auf einem vereinfachten physiologischen Modell basiert.

10. Das System nach einem der Ansprüche 6 bis 8, wobei das Ersatzverfahren das Steuern der Insulininjektionseinrichtung (103) zum Abgeben vorprogrammierter Insulindosen aufweist, die einer dem Patienten verschriebenen Referenz-Basalrate entsprechen.

11. Das System nach einem der Ansprüche 6 bis 8, wobei das Ersatzverfahren das Steuern der Insulininjektionseinrichtung (103) zum Abgeben von Insulindosen aufweist, die durch die Verarbeitungs- und Steuereinheit (105) nach dem durch den Sensor (101) gemessenen aktuellen Blutzuckerspiegel und/oder der Änderungsgeschwindigkeit des durch den Sensor (101) gemessenen Blutzuckers bestimmt werden.

## Claims

1. An automated system of regulation of a patient's blood glucose, comprising:
a blood glucose sensor (101);
an insulin injection device (103); and
a processing and control unit (105),
wherein the processing and control unit (105) is capable of:
a) implementing, taking into account the blood glucose measured by the sensor (101) during a past observation period, a step of automated calibration of a physiological model capable of predicting the evolution of the patient's blood glucose;
b) at the end of the calibration step, calculating at least one numerical indicator representative of the error between the blood glucose estimated from the model and the real blood glucose measured by the sensor over the past observation period;
c) comparing the value of said numerical indicator to predefined thresholds, and determining the duration of a prediction period to come, taking into account the value of the result of said comparison, the duration of the prediction period to come being selected to be all the shorter as the error between the blood glucose estimated from the model and the real blood glucose measured by the sensor over the past observation period is significant, and conversely; and
d) predicting, from the physiological model, the future evolution of the patient's blood glucose over the prediction period to come, and controlling the insulin injection device (103) by taking the prediction into account,
wherein steps a) to d) are repeated at regular intervals,
wherein the processing and control unit (105) is configured to repeat steps (a) to (d) every 1 to 20 minutes, and wherein the future prediction period determined in step (c) is between 1 and 10 hours.

2. The system of claim 1, wherein said at least one numerical indicator comprises the standard deviation between the blood glucose estimated from the model and the real blood glucose measured by the sensor (101) during the past observation period.

3. The system of claim 1 or 2, wherein said at least one numerical indicator comprises the difference between the real blood glucose measured by the sensor (101) and the blood glucose estimated by the model at a given time.

4. The system of any of claims 1 to 3, wherein said at least one numerical indicator comprises the difference between the derivative of the real blood glucose measured by the sensor (101) and the derivative of the blood glucose estimated by the model at a given time.

5. The system of any of claims 1 to 4, wherein the processing and control unit (105) is configured to, at step c), compare the value of said at least one numerical indicator with first thresholds, and select the duration of the prediction period from among a plurality of predefined durations according to the result of the comparison.

6. The system of any of claims 1 to 5, wherein the processing and control unit (105) is further capable, after step b), of determining, based on the value of said at least one numerical indicator, whether the model is sufficiently reliable to be used as a basis for the control of the insulin injection device and, in the opposite case, of controlling the insulin injection device (103) according to a substitute method, without taking into account the prediction performed from the model.

7. The system of claim 6, wherein the processing and control unit (105) is configured to, in order to determine whether the model is sufficiently reliable, compare the value of said at least one numerical indicator with a second threshold.

8. The system of claim 7, wherein the processing and control unit (105) is capable of determining and of automatically adjusting the second threshold from past data measured on the patient, so that the control of the insulin injection device (103) is based on the predictions made by the model at least for a certain percentage of the time.

9. The system of any of claims 6 to 8, wherein the substitute method is a predictive control method based on a simplified physiological model.

10. The system of any of claims 6 to 8, wherein the substitute method comprises controlling the insulin injection device (103) to deliver preprogrammed insulin doses corresponding to a reference basal rate prescribed to the patient.

11. The system of any of claims 6 to 8, wherein the substitute method comprises controlling the insulin injection device (103) to deliver insulin doses determined by the processing and control unit (105) according to the current blood glucose level measured by the sensor (101) and/or to the variation speed of the blood glucose measured by the sensor (101).
